# EUROPEAN PATENT SPECIFICATION

(11) **EP 2 420 244 B1**
(45) Date of publication and mention of the grant of the patent: **06.07.2016**
(21) Application number: 10764387.6
(22) Date of filing: 08.04.2010
(51) Int. Cl.: A61K 38/00, A61P 3/06, A23L 33/18, A61K 38/08

(54) **Lipid metabolism-improving agent**
Mittel zur Förderung des Fettstoffwechsels
Agent améliorant le métabolisme des lipides

(30) Priority: 16.04.2009 JP 2009100147
(43) Date of publication of application: 22.02.2012
(73) Proprietor: Megmilk Snow Brand Co., Ltd., Sapporo-shi Hokkaido 0650043 (JP)
(72) Inventor: SERIZAWA, Atsushi, Sapporo-shi Hokkaido 065-0043 (JP); UETSUJI, Daisuke, Sapporo-shi Hokkaido 065-0043 (JP); HIGURASHI, Satoshi, Sapporo-shi Hokkaido 065-0043 (JP); SAKONO, Masanobu, Miyazaki-shi Miyazaki 889-2192 (JP); FUKUDA, Nobuhiro, Miyazaki-shi Miyazaki 889-2192 (JP)
(74) Representative: Høiberg A/S
(86) International application number: PCT/JP2010/056349
(87) International publication number: WO 2010/119803

(56) References cited:
- WO-A1-2004/069265
- WO-A1-2008/111562
- WO-A2-03/063778
- JP-A- 2008 255 090
- JUNG-YOON PARK ET AL: "Effects of whey protein hydrolysates on lipid profiles and appetite-related hormones in rats fed high fat diet.", JOURNAL OF THE KOREAN SOCIETY OF FOOD SCIENCE AND NUTRITION 2008 CORRESPONDENCE ADDRESS, YEON-SOOK LEE, DEP. OF FOOD & NUTR., SEOUL NAT. UNIV., SEOUL 151-742, KOREA. TEL. 82-2-880-6832. FAX 82-2-884-0305. E-MAIL LYSOOK@SNU.AC.KR, vol. 37, no. 4, 31 December 2008 (2008-12-31), page 428, XP002699078,
- SATOSHI NAGAOKA: 'Whey Peptide no Shishitsu Taisha Kaizen Kino' GEKKAN FUDO KEMIKARU vol. 9, no. 12, 1993, pages 34 - 39

## Description

### TECHNICAL FIELD

We have invented a reagent that has a superior ability to improve lipid metabolism, is less bitter, has excellent stability, and is safe. This reagent can inhibit human and other mammalian adipocytes from incorporating lipids. This application will also describe food, beverages, nutritional supplement, and fodder that comprise this reagent.

### BACKGROUND ART

In recent years, there has been a rise in the risks related to lifestyle diseases, such as obesity, hypertension, and hyperlipidemia and diabetes due to changes in dietary habits, chronic lack of exercise, and excessive stress. When such lifestyle related diseases advance, they can lead to more severe diseases such as arteriosclerosis and myocardial infarction. Of the lifestyle diseases, a large part of changes in dietary habits can be attributed to the shift from the Japanese diet to the western diet, which is mainly composed of meat and has much higher calories compared with the traditional Japanese diet. There are two major ways to prevent lifestyle related diseases caused by high calorie diets. The first method involves decreasing the level of triglycerides and low density lipoprotein cholesterol in the blood, i.e., improving lipid metabolism. The second method involves the inhibition of fat accumulation, i.e., prevention of obesity. Although the two methods are considered to be the same, the actual mechanism behind them differs. There are reagents that improve lipid metabolism, but do not inhibit weight gain (refer to PATENT LITERATURE 1) and reagents that inhibit weight gain but are not associated with lipid metabolism (refer to PATENT LITERATURE 2). The latter method, which inhibits fat accumulation, has received attention for the prevention and treatment of obesity from the aspect of health and beauty. Attempted therapeutic methods include drug and exercise therapy and diet restrictions. Although drug therapy is expected to be effective, there is also a need to consider their side effects. Exercise therapy and diet restrictions accompany temporal or psychological difficulties in terms of compliance in the long term and have low success rates. In addition, the risks of nutrient deficits and anorexia from excessive diet restrictions cannot be ignored. Against such background, there is a need for a simple yet safe reagent or reagent in food and beverages that can be consumed on a daily basis and has ability to improve lipid metabolism.

As a mechanism for improving lipid metabolism, the inhibition of serum cholesterol and triglyceride accumulation can be considered. Of all the substances, soybean peptides from soybean proteins are known to inhibit accumulation of these indices (refer to NON PATENT LITERATURE 1). In addition, hydrolysate from whey protein has been demonstrated to possess lipid metabolism improving ability (refer to

### PATENT LITERATURE 3).

Hydrolysate from milk proteins is used in various products to prevent food allergies from milk and dairy products. Whey protein obtained from cow milk differs from that of breast milk and is believed to be an allergen. To address this issue, enzymatic hydrolysis of whey protein, its method of preparation (refer to PATENT LITERATURE 4 and 5), and the method of denaturation of whey protein by heat and enzymatic hydrolysis by a heat-resistant hydrolase under specific conditions to obtain whey protein hydrolysate has been demonstrated (refer to Patent literature 6).

### CITATION LIST

### PATENT LITERATURE

PATENT LITERATURE 1: JP 2002-226394 A
PATENT LITERATURE 2: JP 2007-308469 A
PATENT LITERATURE 3: JP H05-176713 A
PATENT LITERATURE 4: JP H02-2319 A
PATENT LITERATURE 5: JP H02-138991 A
PATENT LITERATURE 6: JP H04-112753 A
PATENT LITERATURE 7: WO03/063
PATENT LITERATURE 8: WO2008/111562

### NON PATENT LITERATURE

NON PATENT LITERATURE 1: Cho SJ et al. Cholesterol lowering mechanism of soybean protein hydrolysate. J Agric Food Chem. 2007 Dec 26;55(26):10599-604

### SUMMARY OF INVENTION

### TECHNICAL PROBLEM

Whey protein hydrolysate from milk is advantageous in that there is a higher content of branched amino acids compared with soy peptides from soy proteins (PATENT LITERATURE 2), which can produce a better lipid metabolism-improving reagent.

However, a typical whey protein hydrolysate solution is turbid, has limitations when used as an actual product, and is incapable for use as products that are required to be transparent in appearance. In addition, peptides have a characteristic bitter flavor, limiting their use in orally consumed food and fodders.

Although the whey protein hydrolysate has been reported to decrease allergic reactions (PATENT LITERATURE 6), it has not been reported to improve lipid metabolism.

Therefore, the objective of this invention is to produce a lipid metabolism-improving reagent or food, beverage, nutritional supplement, or fodder that contains such reagents and can be consumed as a part of the normal diet. In addition, their application in improving lipid metabolism and preventing or treating metabolic syndrome is not limited, because it is very safe and the aqueous solution is highly transparent and minimally bitter in flavor.

### SOLUTION TO PROBLEM

The preferred embodiments of this invention are shown as follows:
(1) Lipid metabolism-improving reagent comprising whey protein hydrolysate as the active ingredient with the following criteria:
   (i) Molecular weight distribution of 10 kDa or less with main peak between 200 Da and 3 kDa.
   (ii) APL(Average peptide chain length) of 2-8.
   (iii) Free amino acid content of 20% or less.
   (iv) Antigenicity of 1/10,000 or less than that of β-lactoglobulin
(2) The lipid metabolism-improving reagent according to item (1) above characterized in that the whey protein hydrolysate can be obtained by denaturation of whey protein at pH 6-10 and 50°C-70°C and enzymatic hydrolysis by a heat-resistant hydrolase, followed by further heating to inactivate the enzyme.
(3) The lipid metabolism-improving reagent according to item (1) above characterized in that the whey protein hydrolysate can be obtained by the enzymatic hydrolysis of whey protein at pH 6-10 and 20°C-55°C, followed by heating at pH 6-10 and 50°C-70°C for enzymatic hydrolysis of the unhydrolyzed whey protein with a heat-resistant hydrolase while being denatured by heat and further heating to inactivate the enzyme.
(4) Food and beverages comprising the lipid metabolism-improving reagents according to any one of items (1)-(3) above.
(5) Nutritional supplements comprising the lipid metabolism-improving reagents according to any one of items (1)-(3) above.
(6) Fodders comprising the lipid metabolism-improving reagents according to any one of items (1)-(3) above.

Other preferred embodiments of this invention are shown as follows:
(A) improving lipid metabolism, comprising administering whey protein hydrolysate with the following characteristics:
   (i) Molecular weight distribution of 10 kDa or less with main peak between 200 Da and 3 kDa.
   (ii) APL(Average peptide chain length) of 2-8.
   (iii) Free amino acid content of 20% or less.
   (iv) Antigenicity of 1/10,000 or less than that of β-lactoglobulin
(B) The method according to item (A) above characterized in that the whey protein hydrolysate can be obtained by denaturation of whey protein at pH 6-10 and 50°C-70°C and enzymatic hydrolysis by a heat-resistant hydrolase, followed by further heating to inactivate the enzyme.
(C) The method according to item (A) above characterized in that the whey protein hydrolysate can be obtained by the enzymatic hydrolysis of whey protein at pH 6-10 and 20°C-55°C, followed by heating at pH 6-10 and 50°C-70°C for enzymatic hydrolysis of the unhydrolyzed whey protein with a heat-resistant hydrolase while being denatured by heat and further heating to inactivate the enzyme.
(D) inhibiting accumulation of triglycerides, cholesterol, or phospholipids in the serum characterized in that whey protein hydrolysate with the following characteristics is administered:
   (i) Molecular weight distribution of 10 kDa or less with main peak between 200 Da and 3 kDa.
   (ii) APL(Average peptide chain length) of 2-8.
   (iii) Free amino acid content of 20% or less.
   (iv) Antigenicity of 1/10,000 or less than that of β-lactoglobulin
(E) The method according to item (D) above characterized in that the whey protein hydrolysate can be obtained by denaturation of whey protein at pH 6-10 and 50°C-70°C and enzymatic hydrolysis by a heat-resistant hydrolase, followed by further heating to inactivate the enzyme.
(F) The method according to item (D) above characterized in that the whey protein hydrolysate can be obtained by the enzymatic hydrolysis of whey protein at pH 6-10 and 20°C-55°C, followed by heating at pH 6-10 and 50°C-70°C for enzymatic hydrolysis of the unhydrolyzed whey protein with a heat-resistant hydrolase while being denatured by heat and further heating to inactivate the enzyme.
(G) use in preventing and/or treating hyperlipidemia characterized in that whey protein hydrolysate with the following characteristics is administered:
   (i) Molecular weight distribution of 10 kDa or less with main peak between 200 Da and 3 kDa.
   (ii) APL(Average peptide chain length) of 2-8.
   (iii) Free amino acid content of 20% or less.
   (iv) Antigenicity of 1/10,000 or less than that of β-lactoglobulin
(H) The method according to item (G) above characterized in that the whey protein hydrolysate can be obtained by denaturation of whey protein at pH 6-10 and 50°C-70°C and enzymatic hydrolysis by a heat-resistant hydrolase, followed by further heating to inactivate the enzyme.
(I) The method according to item (G) above characterized in that the whey protein hydrolysate can be obtained by the enzymatic hydrolysis of whey protein at pH 6-10 and 20°C-55°C, followed by heating at pH 6-10 and 50°C-70°C for enzymatic hydrolysis of the unhydrolyzed whey protein with a heat-resistant hydrolase while being denatured by heat and further heating to inactivate the enzyme.
(J) use in preventing and/or treating obesity characterized in that whey protein hydrolysate with the following characteristics is administered:
   (i) Molecular weight distribution of 10 kDa or less with main peak between 200 Da and 3 kDa.
   (ii) APL(Average peptide chain length) of 2-8.
   (iii) Free amino acid content of 20% or less.
   (iv) Antigenicity of 1/10,000 or less than that of β-lactoglobulin
(K) The method according to item (J) above characterized in that the whey protein hydrolysate can be obtained by denaturation of whey protein at pH 6-10 and 50°C-70°C and enzymatic hydrolysis by a heat-resistant hydrolase, followed by further heating to inactivate the enzyme.
(L) The method according to item (J) above characterized in that the whey protein hydrolysate can be obtained by the enzymatic hydrolysis of whey protein at pH 6-10 and 20°C-55°C, followed by heating at pH 6-10 and 50°C-70°C for enzymatic hydrolysis of the unhydrolyzed whey protein with a heat-resistant hydrolase while being denatured by heat and further heating to inactivate the enzyme.

### ADVANTAGEOUS EFFECTS OF INVENTION

This lipid metabolism-improving reagent has marked inhibitory effects on triglyceride and total cholesterol accumulation in serum and triglyceride accumulation in the liver. It is effective in the prevention and treatment of diseases such as hyperlipidemia and obesity.

### DESCRIPTION OF EMBODIMENTS

The whey protein hydrolysate mixed with the lipid metabolism-improving reagent can improve lipid metabolism. The hydrolysate can be obtained by denaturing the whey protein at 50°C-70°C and pH 6-10 and enzymatic hydrolysis by a heat-resistant hydrolase, followed by further heating to inactivate the enzyme. Prior to performing the above enzymatic hydrolysis, if the whey protein is hydrolyzed by a protein hydrolase at pH 6-10 and 20-55°C and is not cooled, the yield is considerably higher.

Furthermore, by concentrating the whey protein hydrolysate prepared in this manner using ultrafiltration and/or microfiltration membranes, a stronger lipid metabolism improving effect can be achieved. In addition, such membranes will reduce the bitterness and improve the transparency of the whey protein hydrolysate.

The molecular weight cutoff of the ultrafiltration membrane should be in the range of 1-20 kDa, or preferably 2-10 kDa. Molecular weight cutoff of the microfiltration membrane should be in the range of 100-500 Da, or preferably 150-300 Da.

High transparency is desirable for not limiting the use of this whey protein hydrolysate. Transparency test (described below) should result in an absorbance below 0.014, preferably less than 0.010 or less than 0.005.

The whey protein in this invention was an aggregate, powder, or purified protein derived from the whey of milks obtained from different mammals, such as cows, buffaloes, goats, or humans. In addition, the whey protein enzyme reaction was conducted in aqueous solution conditions.

The pH of the whey protein aqueous solution is usually 6-10; therefore, there is no need to adjust the pH when enzymatic reactions occur. However, in case an adjustment is required, acidic solution, such as hydrochloric acid, citric acid, or lactic acid or alkaline solution, such as sodium hydroxide, calcium hydroxide, or sodium phosphate, can be used to adjust the pH accordingly (pH6-10).

Although this invention described heating at 50-70°C, it is preferable to add the heat-resistant hydrolase prior to heating the whey protein solution and carry out the enzymatic hydrolysis to obtain a higher yield.

Although the commonly used temperatures for proteases are ≤40°C, the optimal temperature for the heat-resistant hydrolase used in this method was ≥45°C. As long as it is a heat-resistant hydrolase known to work optimally at this temperature, any enzyme can be used without restrictions. Some examples of heat-resistant hydrolases are papain, protease S (brand name), proleather (brand name), thermoase (brand name), alcalase (brand name), and protin-A (brand name). In this method, a heat-resistant hydrolase that has about 10% or more residual activity after heating for 30 min at 80°C is desired. In addition, use of multiple enzymes is more effective. The preferred reaction time is 30 min-10 h.

At last, the reaction solution needs to be heated to inactivate the hydrolase. The enzyme that was used in this method can be inactivated by heating the reaction solution for 10 s or more at 100°C and above.

The reaction solution collected as mentioned above was centrifuged and the supernatant was collected. The supernatant is dried to obtain the powdered whey protein hydrolysate product. The precipitate formed during centrifugation has less effect on decreasing allergic reactions compared with that of the supernatant and this should be removed. However, the reaction solution can be dried and used as it is.

The whey protein hydrolysate which can be obtained by this method is quantitated by inhibition ELISA [Japanese Journal of Pediatric Allergy and Clinical Immunology, 1987; 1:12-38 (page 36)].

Its antigenicity was confirmed to be 1/10,000 or less compared with that of β-lactoglobulin and ≤1/10,000 of whey protein and is extremely safe. In addition, because the whey protein hydrolysate solution is transparent, and its bitterness score is approximately 2, there are no limitations to the use of this product. The transparency and the bitterness were assessed by the following methods.

Transparency assessment method: One percent whey protein hydrolysate solution was prepared and absorbance was measured at 650nm.

### Bitterness assessment method:

Ten percent whey protein solution was prepared, and the bitter quinine hydrochloride was added to assess the bitterness. As shown in TABLE 1, if the bitterness score is ≤2, it can be used in foods and beverages.

**[TABLE 1]**

| Quinine hydrochloride concentration | Bitterness score |
|---|---|
| 0.004% | 1 (weak) |
| 0.010% | 2 |
| 0.020% | 3 (strong) |

Although the whey protein hydrolysate can be directly used as a lipid metabolism-improving reagent, it can also be used in powder, granule, tablet, capsule, or solution form in the usual method. Whey protein hydrolysates, which are processed by ultrafiltration or microfiltration, can be directly used as lipid metabolism-improving reagent or they can be used in dried form. In addition, it can be converted into various forms using the usual method.

Furthermore, once formulated, it is also possible to add to nutrients; food and beverages such as yogurt, milk-based beverages, wafers; nutritional supplements; and fodders.

The food and beverages, nutritional supplements, and fodder of the present invention which have lipid metabolism improving effect may mean the whey protein hydrolysate itself. Moreover, they can include a normal constituent, such as stabilizers, sugars, flavors, vitamins, and minerals, flavonoids, and polyphenols.

The food and beverages, nutritional supplements, and fodder of the present invention may be prepared by combining with other commonly used raw materials.

There are no limitations to the use of whey protein hydrolysate in food, beverages, nutritional supplements, and fodders. However, for an adult to orally consume 5 mg or more of whey protein hydrolysate as food, beverages, or fodder, its composition should be 0.001%-10% (w/w) or preferably 0.1%-5% (w/w) of the total mass, depending on the formulation.

Appropriate additives can be added to the active ingredient to produce a preferred formulation of the lipid metabolism-improving reagent and this preparation can be prepared as an oral or non-oral preparation. During the production, diluents and excipients, such as commonly used filler reagents, bulking reagents, bonding and disintegrating agents, surfactants, and lubricants can be used.

Formulation types, including capsules, tablets, granules, powders, solution, suspension, emulsion, suppositories, injections, and ointments, can be used. Examples of excipients include sucrose, lactose, starch, microcrystalline cellulose, mannitol, light anhydrous silicic acid, magnesium aluminate, synthetic alumimum silicate, magnesium, metasilicate aluminate, calcium carbonate, sodium bicarbonate, dibasic calcium phosphate, and carmellose calcium. Two or more of these can be mixed as an additive as well.

The safety of the active ingredient (whey protein hydrolysate) in this lipid metabolism-improving reagent has been confirmed, and as described later, antigenicity has been confirmed at less than 1/10,000 compared with that of β-lactoglobulin and whey protein. Because of the desired factors of transparency and a low degree of bitterness, this aqueous solution has unlimited use as a lipid metabolism-improving reagent, particularly as lipid metabolism-improving reagent that are required to be transparent in appearance.

The following are practical examples and comparisons, as well as experiments, which describe the invention in detail. However, these are solely to provide examples and the use of invention is not limited to these in any way.

### EXAMPLE 1

Five hundred U/g of papain and 50 U/g of proleather (Proleather: Amano Enzymes) was added to 1 L of 10% whey protein solution, pH was adjusted to 8, and heated for 6 h at 55°C to for enzymatic hydrolysis and denaturation of the whey protein. The reaction mixture was then heated to 100°C for 15 s or more to inactivate the enzyme. This solution was centrifuged and the supernatant was collected and dried to obtain the whey protein hydrolysate, which had a molecular weight distribution of 10 kDa or less with a main peak of 1.3 kDa, APL of 7.2, and free amino acid content of 18.9% in the total composition.

Using inhibition ELISA, antigenicity was quantitated to be 1/10,000 or less compared with that of β-lactoglobulin with a yield of 80.3% (after centrifugation of the fluid in which the enzyme reaction occurred, yield was calculated by dividing the dry weight of the total reaction mixture by the dry weight of the supernatant) and bitterness score of 2.

Transparency test results showed that the solution was highly transparent with an absorbance of 0.008 at 650 nm.

The whey protein hydrolysate obtained in this manner can be directly used as a lipid metabolism-improving reagent.

### EXAMPLE 2

Five hundred U/g of papain and 50 U/g of proleather (Proleather:Amano Enzymes) was added to 1 L of 10% whey protein solution, adjusted to pH 8, and heated for 3 h at 50°C for enzymatic hydrolysis. This reaction mixture was then heated to 55°C and incubated for 3 h at this temperature for enzymatic hydrolysis along with heat denaturation. The enzyme was inactivated by heating to 100°C for 15 s or more. This reaction mixture was processed in UF (STC) membrane with a molecular weight cutoff of 10 kDa and MF (STC) with molecular weight cutoff of 300 Da. The concentrated fraction was collected and dried to obtain the whey protein hydrolysate.

The molecular weight distribution of the obtained whey protein hydrolysate was 10 kDa or less with main peak of 500 Da, APL of 3.0, and free amino acid content of 15.2% in total composition.

Inhibition ELISA showed an antigenicity of 1/10,000 or less than that of β-lactoglobulin, a yield of 65.4%, and bitterness score of 2.

Transparency test results showed that the solution was highly transparent with an absorbance of 0.004 at 650nm.

The whey protein hydrolysate obtained using this method can be directly used as a lipid metabolism-improving reagent.

### [Test Example 1]

### (Animal experiment)

Four-week-old Sprague-Dawley male rats (Kyudo) were pre-fed for 7 days with commercial powder fodder CE-2 (Japan CLEA) and divided into two groups: casein and soy peptide group (HI-NUTE AM, Fuji oil) as control group and whey protein hydrolysate group (whey protein hydrolysate described in Practical example 1). They were freely fed the fodder prepared according to the AIN-76 composition shown in TABLE 2 for two weeks. Each measurement was taken after the administration.

The serum cholesterol, triglyceride, and phospholipid levels were measured using the commercial enzyme kits: Cholesterol E-test Wako, Triglyceride E-test Wako, and Phospholipid C-test Wako. All data are shown as mean ± standard error (SE). Statistical analysis was performed using the Tukey-Kramer multiple comparison test and a P < 0.05 was considered statistically significant.

**[TABLE 2]**

| **Fodder Composition (%)** | | | |
|---|---|---|---|
| | **Control group** | **Soy peptide group** | **Whey protein hydrolysate group** |
| **Casein** | 20 | - | - |
| **Soy peptide** | - | 20 | - |
| **Whey Protein hydrolysate** | - | - | 20 |
| ***β* - cornstarch** | 15 | 15 | 15 |
| **Cellulose** | 5 | 5 | 5 |
| **Corn oil** | 5 | 5 | 5 |
| **Methionine** | 0.3 | 0.3 | 0.3 |
| **Mineral mixture** | 3.5 | 3.5 | 3.5 |
| **Vitamin mixture** | 1 | 1 | 1 |
| **Choline bitartrate** | 0.2 | 0.2 | 0.2 |
| **Sucrose** | **Total mass of 100** | **Total mass of 100** | **Total mass of 100** |

**[TABLE 3]**

| **Growth parameters and various organ weights of rats** | | | |
|---|---|---|---|
| | **Control group** | **Soy peptide group** | **Whey protein hydrolysate group** |
| **Initial BW (g)** | 161 ± 3 | 161 ± 3 | 159 ± 2 |
| **Final BW (g)** | 288 ± 6^{ab} | 295 ± 6^{b} | 271 ± 6^{a} |
| **BW gain (g/day)** | 9.07 ± 0.26^{ab} | 9. 55 ± 0.32^{b} | 8.07 ± 0.32^{a} |
| **Food Cons. (g/day)** | 23.6 ± 0.5 | 23.6 ± 0.7 | 21. 9 ± 0.6 |
| **Liver W (g/100 g BW)** | 5. 78 ± 0.12 | 5.38 ± 0. 19 | 5.89 ± 0.16 |
| **Adipose TW (g/100 g BW)** | | | |
| **- Periadrenal fat** | 1. 45 ± 0.19 | 1.29 ± 0.10 | 1. 21 ± 0.14 |
| **- Epididymal fat** | 1.14 ± 0.04 | 1.01 ± 0.04 | 1.07 ± 0.06 |
| **Spleen W (g/100 g BW)** | 0.303 ± 0.017^{ab} | 0.266 ± 0.005^{a} | 0. 332 ± 0.016^{b} |
| **Kidney W (g/100 g BW)** | 0.851 ± 0.020^{a} | 0.861 ± 0.017^{ab} | 0.936 ± 0.024^{b} |
| **Heart W (g/100 g BW)** | 0.391 ± 0.005 | 0.420 ± 0.015 | 0.406 ± 0. 012 |
| **Lung W (g/100 g BW)** | 0.446 ± 0.009 | 0.418 ± 0.015 | 0.460 ± 0.014 |

| | | | |
|---|---|---|---|
| MEANS±SE(n=6-7) **ab: significant difference between different letters (P < 0.05)** **BW: body weight** **Food Cons.: Food Consumption** **W: weight** **TW: tissue weight** | | | |

**[TABLE 4]**

| **Rat Serum Lipid Concentration** | | | |
|---|---|---|---|
| | **Control group** | **Soy peptide group** | **Whey protein hydrolysate group** |
| **Serum lipid conc. (mg/dL)** | | | |
| **Triglyceride** | 293 ± 28 | 248 ± 20 | 256 ± 35 |
| **Cholesterol** | 113 ± 5 | 92.7 ± 4.8 | 88.0 ± 9.0 |
| **Phospholipid** | 254 ± 8 | 222 ± 9 | 211 ± 16 |

| | | | |
|---|---|---|---|
| MEANS ± SE (n=6-7) | | | |

TABLE 3 shows the values of body, organ, adipose tissue weights, and food consumption.

Final body weight and body weight gain showed a larger value in the soy peptide group compared with that of the control group and showed a smaller value in the whey protein hydrolysate group. The final results showed a significant difference between the soy peptide group and the whey protein hydrolysate group. When the 3 groups were compared, food consumption did not show a significant difference, yet a slightly smaller value was observed for the whey protein hydrolysate group compared with that of the control and soy peptide groups. Similarly, liver weight, measured per 100 g of body weight, showed a slightly lower value in the soy peptide group compared with that of the control group, however, there was no significant difference. Kidney weight was measured per 100 g of body weight and showed a significantly large value in the whey protein hydrolysate group compared with that of the control group. On comparing the group with respect to the heart and lung weights (per 100 g of body weight), no major difference was observed.

TABLE 4 shows serum lipid concentration.

The results show that the serum triglyceride concentration in the soy peptide group and whey protein hydrolysate group is approximately 15% lower compared with the control group.

Serum cholesterol level was lower (approximately 20%) in the soy peptide and whey protein hydrolysate groups compared with that of the control group.

Serum phospholipid and triglyceride concentrations had similar results, and the whey protein hydrolysate group showed a smaller value compared with that of the soy peptide group.

Therefore, improvement of serum cholesterol metabolism was more effective in the whey protein hydrolysate group compared with that of the soy peptide group.

Furthermore, whey protein hydrolysate solution is transparent and its bitterness score is as low as 2; therefore, there is no limitation in its use in manufactured products. Thus, there is a clear effect of suppressing accumulation of serum triglyceride, cholesterol, and phospholipid.

### EXAMPLE 3

### (Preparation of the tablet form)

The ingredients listed in TABLE 5 were mixed with 100 mg of whey protein hydrolysate and compressed by the usual method to produce 1-g tablets.

**[TABLE 5]**

| | |
|---|---|
| **Dextrose monohydrate** | 83.5 **(weight %**) |
| **Whey protein hydrolysate obtained in EXAMPLE 1** | 10.0 |
| **Mineral mixture** | 5.0 |
| **Sugar ester** | 1.0 |
| **Flavor** | 0.5 |

| | |
|---|---|
| **The ingredients listed were mixed with 100 mg of whey protein hydrolysate to produce 1-g tablets.** | |

### EXAMPLE 4

### (Preparation of the nutritional supplement)

In 4950 g of deionized water, 50 g of whey protein hydrolysate obtained from EXAMPLE 2 was dissolved, heated to 50°C, and mixed for 30 min at 6000 rpm using a TK homomixer (TK ROBO MICS; Tokushu Kika Kogyo) to obtain a whey protein hydrolysate solution with a whey protein concentration of 50 g/5 kg. The following ingredients were added to 5.0 kg of the whey protein hydrolysate solution: 5.0kg of casein, 5.0 kg of soy protein, 1.0 kg of fish oil, 3.0 kg of perilla oil, 18.0 kg of dextrin, 6.0 kg of minerals, 1.95 kg of vitamins, 2.0 kg of emulsifier, 4.0 kg of stabilizer, and 0.05 kg of flavor. This mixture was filled into a 200 mL retort pouch and sterilized for 20 min at 121°C with a retort sterilizer (First pressure vessel; TYPE: RCS-4CRTGN; Hisaka works) to produce 50 kg of nutritional supplement.

The amount of whey protein hydrolysate in 100 g of the nutritional supplement was 100 mg.

### EXAMPLE 5

### (Preparation of beverages)

In 409 g of deionized water, 300 g of skim milk was dissolved, and the whey protein hydrolysate obtained in EXAMPLE 1 was mixed in this mixture, heated to 50°C, and stirred with the ultradisperser (Ultra-Turrax T-25; IKA Japan) for 30 min at 9500 rpm. The following ingredients were added to 166 g of deionized water: 100 g of maltitol, 2 g of acidulant, 20 g of reduced sugar syrup, and 2 g of flavor packed in 100 ml glass bottles, sterilized for 15 min at 90°C, and sealed tightly to prepare ten 100 ml bottled beverages.

The amount of whey protein hydrolysate in 100 ml of this beverage was 100 g.

### EXAMPLE 6

### (Preparation of dog food)

In 99.8 kg of deionized water, 200 g of the whey protein hydrolysate obtained from EXAMPLE 2 was dissolved, heated to 50°C, and stirred for 40 min at 3600 rpm using TK homomixer (MARK II 160 type: Tokushu Kika Kogyo) to obtain whey protein hydrolysate solution with a whey protein concentration of 2 g/100 g. The following ingredients were added to 10 kg of whey protein hydrolysate solution: 12 kg of soy bean flour (soybean meal), 14 kg of skim milk, 4 kg of soy oil, 2 kg of corn oil, 23.2 kg of palm oil, 14 kg of corn starch, 9 kg of wheat flour, 2 kg of wheat bran, 5 kg of vitamins, 2.8 kg of cellulose, and 2 kg of minerals. This mixture was sterilized for 4 min at 120°C to produce 100 kg of dog food.

The amount of whey protein hydrolysate in 100 g of dog food was 20 mg.

## Claims

1. A composition comprising whey protein hydrolysate, wherein said whey protein hydrolysate has
(1) a molecular weight distribution of 10 kDa or less with main peak between 200 Da and 3 kDa,
(2) an APL (Average peptide chain length) of 2-8,
(3) a free amino acid content of 20% or less, and
(4) an antigenicity of 1/10,000 or less than that of β-lactoglobulin, for use in the prevention and/or treatment of metabolic syndrome.

2. The composition for use according to claim 1, wherein said whey protein hydrolysate is obtained by denaturation of whey protein at pH 6-10 and 50°C-70°C and enzymatic hydrolysis by a heat-resistant hydrolase, followed by further heating to inactivate the heat-resistant hydrolase.

3. The composition for use according to claim 1, wherein said whey protein hydrolysate is obtained by enzymatic hydrolysis of whey protein at pH 6-10 and 20°C-55°C, followed by heating at pH 6-10 and 50°C-70°C for enzymatic hydrolysis of the unhydrolyzed whey protein with a heat-resistant hydrolase while being denatured by heat and further heating to inactivate the enzyme.

4. The composition for use according to any of claims 1-3, wherein said composition is a food, a beverage, a nutritional supplement or a fodder comprising said whey protein hydrolysate.

5. The composition for use according to any of claims 1-4, wherein said composition is in powder, granule, tablet, capsule, solution, suspension or dried form.

6. The composition for use according to any of claims 1-4, wherein said whey protein hydrolysate is further processed by ultrafiltration or microfiltration.

7. The composition for use according to any of claims 1-4, wherein said whey protein hydrolysate is able to improve lipid metabolism.

8. The composition for use according to any of claims 1-5, wherein said whey protein hydrolysate inhibits accumulation of triglycerides, cholesterol, and/or phospholipids in serum.

9. The composition for use according to any of claims 1-6, wherein hyperlipidemia is prevented or treated.

10. The composition for use according to any of claims 1-6, wherein obesity is prevented or treated.

## Patentansprüche

1. Zusammensetzung, umfassend Molkenproteinhydrolysat, wobei das Molkenproteinhydrolysat
(1) eine Molekulargewichtsverteilung von 10 kDa oder weniger mit einem Hauptpeak zwischen 200 Da und 3 kDa,
(2) eine APL (average peptide chain length; mittlere Peptidkettenlänge) von 2-8,
(3) einen Gehalt an freien Aminosäuren von 20 % oder weniger und
(4) eine Antigenität von 1/10.000 oder weniger als diejenige von β-Lactoglobulin
aufweist, zur Verwendung bei der Vorbeugung und/oder Behandlung von metabolischem Syndrom.

2. Zusammensetzung zur Verwendung nach Anspruch 1, wobei das Molkenproteinhydrolysat durch Denaturierung von Molkenprotein bei pH 6 - 10 und 50 °C - 70 °C und enzymatische Hydrolyse durch eine hitzebeständige Hydrolase, gefolgt von weiterem Erhitzen zum Inaktivieren der hitzebeständigen Hydrolase, erhalten wird.

3. Zusammensetzung zur Verwendung nach Anspruch 1, wobei das Molkenproteinhydrolysat durch enzymatische Hydrolyse von Molkenprotein bei pH 6 - 10 und 20 °C - 55 °C, gefolgt von Erhitzen bei pH 6 - 10 und 50 °C - 70 °C zur enzymatischen Hydrolyse des unhydrolysierten Molkenproteins mit einer hitzebeständigen Hydrolase während der Denaturierung durch Hitze und weiterem Erhitzen zum Inaktivieren des Enzyms, erhalten wird.

4. Zusammensetzung zur Verwendung nach einem der Ansprüche 1 - 3, wobei die Zusammensetzung ein Nahrungsmittel, ein Getränk, ein Nahrungsergänzungsmittel oder ein Futtermittel ist, welches das Molkenproteinhydrolysat umfasst.

5. Zusammensetzung zur Verwendung nach einem der Ansprüche 1 - 4, wobei die Zusammensetzung in Pulver-, Granulat-, Tabletten-, Kapsel-, Lösungs-, Suspensions- oder Trockenform vorliegt.

6. Zusammensetzung zur Verwendung nach einem der Ansprüche 1 - 4, wobei das Molkenproteinhydrolysat durch Ultrafiltration oder Mikrofiltration weiterverarbeitet wird.

7. Zusammensetzung zur Verwendung nach einem der Ansprüche 1 - 4, wobei das Molkenproteinhydrolysat in der Lage ist, den Lipidstoffwechsel zu verbessern.

8. Zusammensetzung zur Verwendung nach einem der Ansprüche 1 - 5, wobei das Molkenproteinhydrolysat die Serumtriglycerid-, -cholesterin- und/oder -phospholipidakkumulation hemmt.

9. Zusammensetzung zur Verwendung nach einem der Ansprüche 1 - 6, wobei der Hyperlipidämie vorgebeugt oder diese behandelt wird.

10. Zusammensetzung zur Verwendung nach einem der Ansprüche 1 - 6, wobei der Fettsucht vorgebeugt oder diese behandelt wird.

## Revendications

1. Composition comprenant un hydrolysat de protéine de lactosérum, dans laquelle ledit hydrolysat de protéine de lactosérum a
(1) une distribution du poids moléculaire de 10 kDa ou moins avec un pic principal entre 200 Da et 3 kDa,
(2) une APL (longueur moyenne de chaîne peptidique) de 2 à 8,
(3) une teneur en acides aminés libres de 20 % ou moins, et
(4) une antigénicité de 1/10 OOOème ou moins de celle de la β-lactoglobuline, pour son utilisation dans la prévention et/ou le traitement du syndrome métabolique.

2. Composition pour son utilisation selon la revendication 1, dans laquelle ledit hydrolysat de protéine de lactosérum est obtenu par dénaturation de la protéine de lactosérum à pH 6 à 10 et à 50 °C à 70 °C et hydrolyse enzymatique par une hydrolase thermo-résistante, suivie par un chauffage supplémentaire pour inactiver l'hydrolase thermo-résistante.

3. Composition pour son utilisation selon la revendication 1, dans laquelle ledit hydrolysat de protéine de lactosérum est obtenu par hydrolyse enzymatique de la protéine de lactosérum à pH 6 à 10 et à 20 °C à 55 °C, suivie par le chauffage à pH 6 à 10 et à 50 °C à 70 °C pour l'hydrolyse enzymatique de la protéine de lactosérum non hydrolysée avec une hydrolase thermo-résistante alors qu'elle est dénaturée par la chaleur et un chauffage supplémentaire pour inactiver l'enzyme.

4. Composition pour son utilisation selon l'une quelconque des revendications 1 à 3, dans laquelle ladite composition est un aliment, une boisson, un complément alimentaire ou un fourrage comprenant ledit hydrolysat de protéine de lactosérum.

5. Composition pour son utilisation selon l'une quelconque des revendications 1 à 4, dans laquelle ladite composition est sous la forme d'une poudre, d'un granule, d'un comprimé, d'une gélule, d'une solution, d'une suspension ou sous forme anhydre.

6. Composition pour son utilisation selon l'une quelconque des revendications 1 à 4, dans laquelle ledit hydrolysat de protéine de lactosérum est en outre traité par ultrafiltration ou microfiltration.

7. Composition pour son utilisation selon l'une quelconque des revendications 1 à 4, dans laquelle ledit hydrolysat de protéine de lactosérum est capable d'améliorer le métabolisme des lipides.

8. Composition pour son utilisation selon l'une quelconque des revendications 1 à 5, dans laquelle ledit hydrolysat de protéine de lactosérum inhibe l'accumulation des triglycérides, du cholestérol et/ou des phospholipides dans le sérum.

9. Composition pour son utilisation selon l'une quelconque des revendications 1 à 6, dans laquelle l'hyperlipidémie fait l'objet d'une prévention ou d'un traitement.

10. Composition pour son utilisation selon l'une quelconque des revendications 1 à 6, dans laquelle l'obésité fait l'objet d'une prévention ou d'un traitement.
